# EUROPEAN PATENT APPLICATION

(11) **EP 4 759 236 A1**
(43) Date of publication of application: **17.06.2026**
(21) Application number: 24219781.2
(22) Date of filing: 13.12.2024
(51) Int. Cl.: A61B 6/00

(54) **X-RAY RADIOGRAPHY APPARATUS**

(71) Applicant: Koninklijke Philips N.V., 5656 AE Eindhoven (NL)
(72) Inventor: WEISS, Steffen, Eindhoven (NL)
(74) Representative: Philips Intellectual Property & Standards

(57) **Abstract**

The subject-matter of the present disclosure relates to an x-ray radiography 'XR' apparatus (100). The apparatus comprises a first image sensor (104) configured to capture an X-ray image of a target area (106), a second image sensor (114) arranged with a field of view comprising the target area in a first direction (116), and a mirror (118) disposed within the field of view of the second image sensor (114), and arranged at an angle to provide the second image sensor (114) with a view of the target area (106) from a second direction (120) at least partly orthogonal to the first direction (116).

## Description

### FIELD

The subject-matter of the present disclosure relates to an x-ray radiography 'XR' apparatus; in particular, a digital XR apparatus. Yet more specifically, the present disclosure relates to an XR apparatus which may allow for respiratory based triggering of an XR camera.

### BACKGROUND

Many X-ray scans, such as those performed at a wall stand, must be done in full inhalation. Current XR systems are not equipped with any respiratory triggering mechanism, but staff have to give breathing commands to the patient via the patient intercom. Staff have no means to check for the correct breathing state of the patient when the exposure is done. This manual procedure requires time and attention by staff and patient, and it is prone to error for non-compliant patients leading to compromised image quality and unnecessary X-ray exposure.

It is therefore now desired to provide an alternative XR apparatus which improves upon the prior art.

### SUMMARY

Most DXR systems already have a camera integrated at the X-ray tube, viewing the patient and used for correct positioning in front of the detector. In an embodiment, it is proposed to use this camera for respiratory triggering of the exposure. Respiratory triggering of a camera has been previously shown to be possible using e.g., Philips VitalEye for camera-based respiratory triggering of MR scans. In short, the algorithm detects parts of the image that present respiratory motion and generates a live respiratory curve and respiratory triggers at full inspiration or expiration.

However, since in current XR systems the direct camera view does not provide any large respiratory motion components, an angulated mirror may be added to translate the respiratory motion of the chest wall into motion visible in the camera view. Image parts with this motion may be automatically detected by the triggering algorithm, translated into a breathing curve, and used to trigger the exposure as soon as a breath-hold in inhalation has been achieved by the patient.

If used with automatic breathing commands, the entire workflow may become fully automatic and avoid any false exposures.

Suitably, according to a first aspect of the present invention, there is provided an x-ray radiography 'XR' apparatus. The apparatus comprises a first image sensor configured to capture an X-ray image of a target area, a second image sensor arranged with a field of view comprising the target area in a first direction, and a mirror disposed within the field of view of the second image sensor, and arranged at an angle to provide the second image sensor with a view of the target area from a second direction at least partly orthogonal to the first direction. Thus a targets respiratory motion may be readily observed using the second image sensor, and control of the XR apparatus based thereon.

In an example, the second image sensor may be configured to capture images to position a target in the target area.

In an example, the field of view in the first direction may be substantially parallel with a field of view of the first image sensor.

In an example, the mirror may be integrated with one of the first image sensor and an X-ray generator which exposes the target area with X-ray radiation to be captured by the first image sensor.

In an example, the mirror may be positioned to reflect light from the first direction into approximately the second direction.

In an example, the mirror may be a dielectric mirror.

In an example, the second image sensor may be configured to capture images in the infra-red spectrum.

In an example, the apparatus further comprises means to illuminate the target with infra-red light. Such means may be comprised in/on the mirror.

In an example, the mirror may be curved in at least one dimension to improve the field of view in the second direction.

In an example, the mirror may comprise an optical marker for indicating the position of the mirror in an image captured by the second image sensor.

In an example, the apparatus comprises at least one processor configured to analyze, in real time, a plurality of images captured by the second image sensor to determine a respiratory pattern of the target and trigger the XR apparatus to capture an image with the first image sensor based on the determined respiratory pattern. That is, the apparatus may be configured for automatic triggering of the x-ray capture based on respiratory motion observable by the second image sensor as a result of the positioning of the mirror.

In an example, analyzing the respiratory pattern comprises determining maxima and/or minima of the respiratory pattern, and triggering the first image sensor is based on determining that the respiratory pattern is currently indicating a maximum and/or minimum, respectively.

In an example, there may be provided a display configured to output an image captured by the first image sensor.

These and other aspects of the present invention will be apparent from and elucidated with reference to the embodiment(s) described hereinafter.

### BRIEF DESCRIPTION OF DRAWINGS

The embodiments of the present inventions may be best understood with reference to the accompanying Figures, in which:
Fig. 1 shows a schematic of an example apparatus;
Fig. 2 shows an example wall stand apparatus;
Fig. 3 shows (a part of) another example wall stand apparatus;
Fig. 4 shows another example apparatus;
Fig. 5 shows example respiratory pattern analysis.

### DESCRIPTION OF EMBODIMENTS

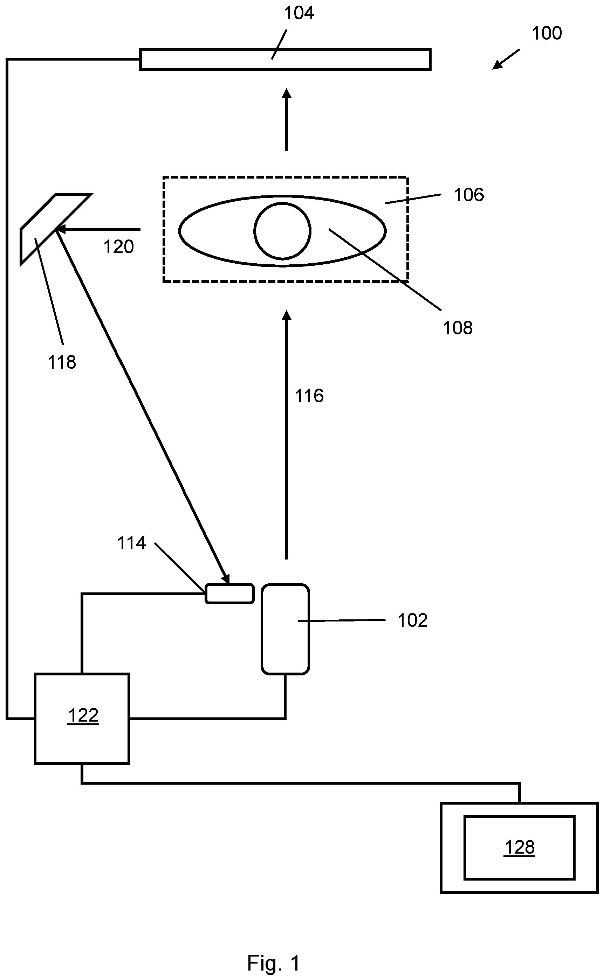

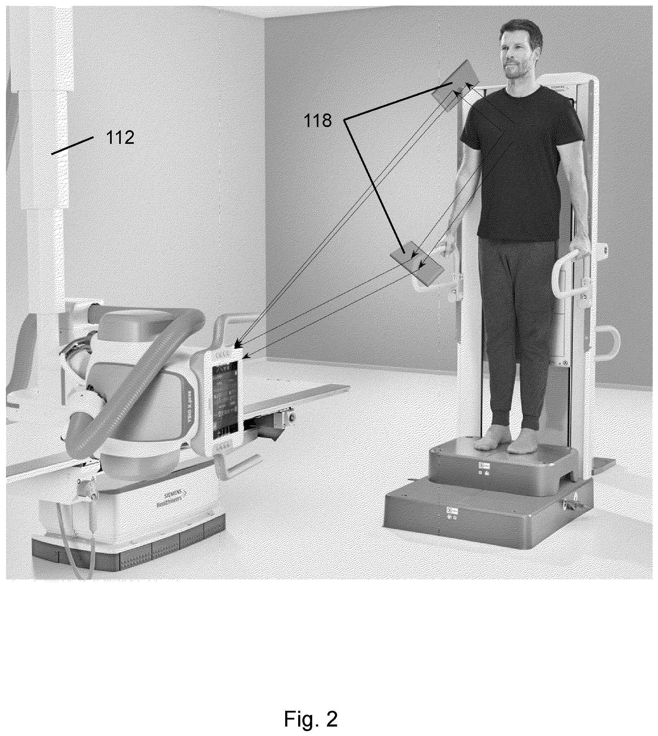

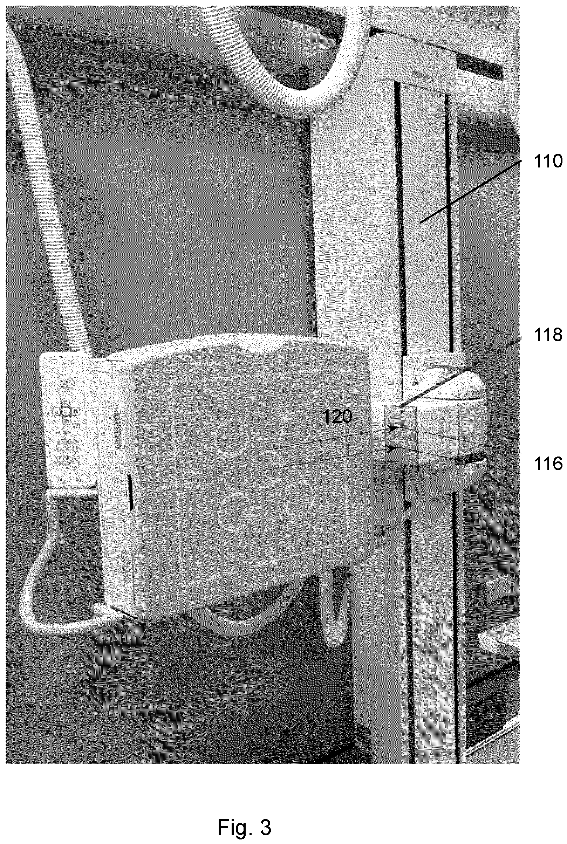

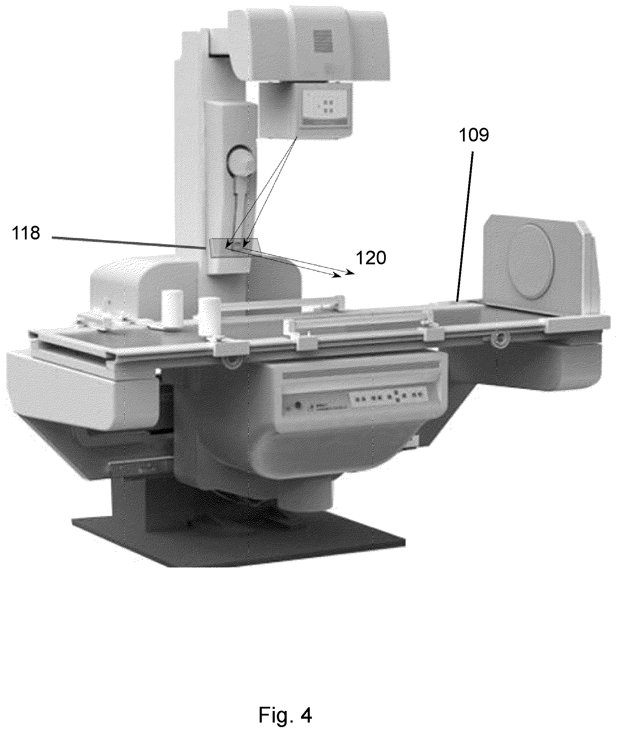

With reference to Figs 1 to 4, there is shown an X-ray radiography 'XR' apparatus 100. In the present examples, the XR apparatus 100 may be configured in a wall stand type arrangement (i.e., horizontal) such as in Figs 2 and 3, or a bed type arrangement (i.e., horizontal) such as in Fig. 4, although the disclosure is not limited thereto.

Suitably, the apparatus 100 comprises radiographic imaging means; for example, comprising a radiation generator 102 and a radiographic image sensor 104, also termed a first image sensor 104 herein. In particular, the radiation generator 102 may be configured to generate X-rays and the first image sensor 104 correspondingly configured to capture an X-ray image. As will be familiar to those in the art, the arrangement of the radiographic imaging means 102, 104 defines a target area 106 in which a target 108, such as a patient, or part thereof, may be positioned in order to capture a radiographic image based on the penetration of radiation through the target 108 in the target area 106. Put another way, it may be considered that the first image sensor 104 is configured to capture an (X-ray) image of the target area 106.

In some examples, the radiographic imaging means 102, 104 are provided in a fixed arrangement, with the target 108 being moved with respect to the imaging means 102, 104 to allow for imaging of different parts of the target 108. Accordingly, the target area 106 to be imaged may be considered fixed. For example, as shown in Fig. 4, a bed 109 on which the target rests may be configured to move the target 108 horizontally in between the generator 102 and sensor 104.

In other examples, the radiographic imaging means 102, 104 may be coupled to suitable movement means such that one or both of the first imaging sensor 104 and radiation generator 102 may be moved relative to the target 108. That is, the target 108 may be considered substantially fixed in position, while the target area 106 to be imaged may be re-positioned according to the moveable arrangement between the first imaging sensor 104 and radiation generator 102. For example, as shown in Figs 2 and 3, the imaging sensor 104 may be coupled to one or more rails 110 to provide lateral motion, while the generator 102 may be coupled to a moveable arm 112.

In order to help position the target 108, the apparatus 100 may comprise a second image sensor 114 having a field of view which encompasses the target area 106. That is, the second image sensor 114 may be provided on the apparatus 100 to capture images of the target 108 within the target area 106, from which an operator may instruct adjustment of the target 108 and/or apparatus 100 in order to allow for imaging of the correct part of the target 108. That is, the images captured by the second image sensor allow for accurate alignment of the radiographic imaging means without exposure of the target to radiation.

More specifically, the second image sensor 114 may be considered to have a field of view corresponding to a first direction 116. The field of view in the first direction 116 may be substantially parallel with, or aligned with, the field of view of the first image sensor. That is, the first direction 116 may be parallel with a nominal axial line connecting the first image sensor 104 and the radiation generator 102. Put yet another way, the field of view in the first direction may be aligned with the imaging angle of the radiographic imaging means.

The second imaging sensor 114 may be configured to capture light at any suitably wavelength which allows for positioning the target 108; preferably of course the second image sensor may be configured to capture visible light.

In some examples, the second image sensor 114 is integrated as part of the radiographic imaging means. That is, as a component of the respective part of the imaging means, whether that's the first imaging sensor 104, the x-ray generator 102, or another related component thereof. In this way, the alignment of the second imaging sensor 114 with respect to the first imaging sensor 104 and target area 106 may be assured.

The apparatus 100 further comprises a mirror 118 disposed within the field of view of the second imaging apparatus 114. The mirror 118 is arranged at an angle in order to provide the second image sensor 114 with a second field of view of the target area 106. More specifically, the mirror 118 provides the second image sensor 114 with a view of the target area 106 from a second direction 120. The second direction may be at least partly orthogonal to the first direction 116. That is, the field of view in the second 120 corresponds to a field of view substantially orthogonal to the field of view according to the first direction 116. Put yet another way, the mirror 118 is positioned to reflect light from the first direction 116 into approximately the second direction 120. In this way, the mirror 118 allows the second image sensor 114 to capture images of the target at an angle different to the radiographic imaging angle. Suitably, where the target 108 is a patient, and substantially positioned face on to the radiographic imaging means, the mirror 116 allows the second image sensor to image the patients chest from an angle at from which chest motion can be observed.

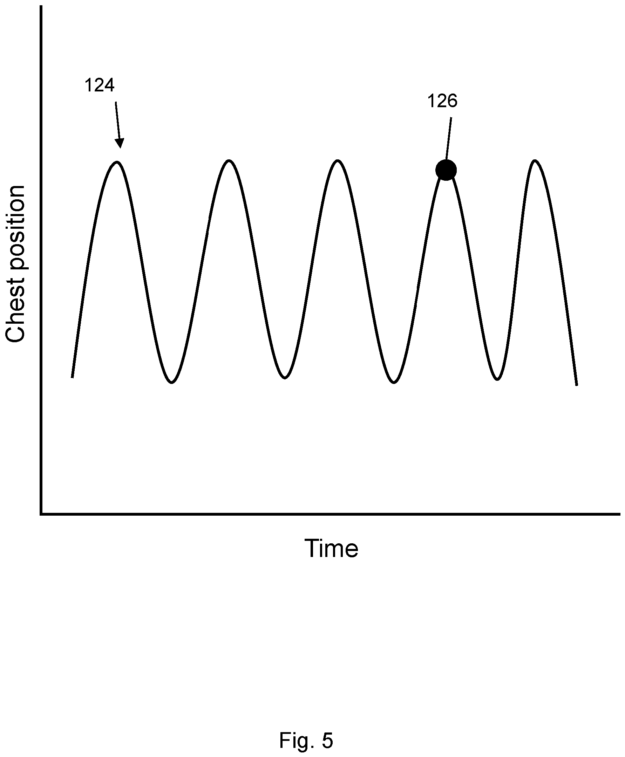

Suitably, the XR apparatus 100 may comprise at least one processor 122 configured to analyze, in real time, images captured by the second imaging sensor 114. More specifically, the second image sensor 114 may be configured to capture a video of the patient 108. The processor 122 analyses the captured images to determine a respiratory pattern 124 of the target 108 as shown for example by Fig. 5.

Based on the determined respiratory pattern, the processor then controls operation of the radiographic imaging means 102, 104. That is, the processor 122 controls the generator 102 to expose the target 108 to radiation, while the first imaging means 104 then captures an image of that radiation. Put another way, the processor 122 is configured to trigger the first image sensor 104 to capture an image, based on the analysis of the respiratory pattern.

More specifically, determining the respiratory pattern may comprise determining maxima and/or minima in the pattern corresponding to the target 108 being in full inhalation or full exhalation. It will be appreciated that breath hold (with full or empty lungs) is often a criteria patients are asked to meet while having radiographs taken.

Analysis of the respiratory pattern 124 is ongoing in real time, such that when it is determined that the pattern is at a current maximum or minimum (such as maximum 126) then the processor triggers the radiographic image. Suitably, it will be appreciated that the image captured by the first image sensor 104, as a result of the triggering, may then be displayed on a display 128 (which may be considered a component part of the apparatus 100, or coupled to the apparatus 100).

In some examples the mirror 118 may be dielectric. That is, comprising a surface comprising a plurality of layers formed from dielectric material. In this way the mirror 118 may be configured to reflect infrared light. Suitably, the second imaging means 114 may be correspondingly configured to capture infra-red light, for example by using a filter.

Suitably, the mirror 118 may be provided with means to illuminate the target area 106 (and therefore target 108) with infrared light. It will also be appreciated that such means may be provided elsewhere on the apparatus 100, for example proximate to the second image sensor 114.

To improve visibility of the target area in the second direction 120, in some examples the mirror 118 may be curved in at least one dimension; e.g., by being convex. Also, the mirror 118 may be positioned in an area determined to be suitably removed from target area 106 so as less likely to be interfered with by the target 108. For example, the mirror 118 may be arranged above a shoulder position of the target 108 (see e.g., Fig. 2).

It will also be appreciated that the field of view of the target 108 in the second direction corresponds only to a small area of the full image captured by the second image sensor 114. In order to help the analysis of the respiratory pattern, the mirror 118 may be provided with an optical marker by which the analysis software may identify a position of the mirror (and therefore image corresponding to the second field of view) within the image by the second image sensor 114. In one example the optical marker may be applied to the entire boundary to the mirror 114 so that, in effect, the image captured by the second image sensor 114 comprises a bounding box corresponding to the field of view in the second direction 120.

In summary, exemplary embodiments of an improved XR apparatus have been described. The described embodiments address problems associated with current techniques for XR radiography. Moreover, the described embodiments may be realized as a retrofit for many existing XR systems (e.g., by providing a clip on mirror and a software update).

While the invention has been illustrated and described in detail in the drawings and foregoing description, such illustration and description are to be considered illustrative or exemplary and not restrictive; the invention is not limited to the disclosed embodiments.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measured cannot be used to advantage. Any reference signs in the claims should not be construed as limiting the scope.

## Claims

1. An x-ray radiography 'XR' apparatus (100), comprising:
a first image sensor (104) configured to capture an X-ray image of a target area (106);
a second image sensor (114) arranged with a field of view comprising the target area in a first direction (116);
a mirror (118) disposed within the field of view of the second image sensor, and arranged at an angle to provide the second image sensor with a view of the target area from a second direction (120) at least partly orthogonal to the first direction.

2. The XR apparatus of claim 1, wherein the second image sensor is configured to capture images to position a target (108) in the target area.

3. The XR apparatus of claim 1 or 2, wherein the field of view in the first direction is substantially parallel with a field of view of the first image sensor.

4. The XR apparatus of any preceding claim, wherein the mirror is integrated with one of the first image sensor and an X-ray generator which exposes the target area with X-ray radiation to be capture by the first image sensor.

5. The XR apparatus of any preceding claim, wherein the mirror is positioned to reflect light from the first direction into approximately the second direction.

6. The XR apparatus of any preceding claim, wherein the mirror is a dielectric mirror.

7. The XR apparatus of claim 6, wherein the second image sensor is configured to capture images in the infra-red spectrum.

8. The XR apparatus of claim 7, further comprising means to illuminate the target with infra-red light.

9. The XR apparatus of claim 8, wherein the mirror comprises the means to illuminate the target.

10. The XR apparatus of any preceding claim, wherein the mirror is curved in at least one dimension.

11. The XR apparatus of any preceding claim, wherein the mirror comprises an optical marker for indicating the position of the mirror in an image captured by the second image sensor.

12. The XR apparatus of any preceding claim, further comprising at least one processor (122) configured to:
analyze, in real time, a plurality of images captured by the second image sensor to determine a respiratory pattern (124) of the target; and
trigger the first image sensor to capture an image based on the determined respiratory pattern.

13. The XR apparatus of claim 12, wherein analyzing the respiratory pattern comprises determining maxima of the respiratory pattern, and triggering the first image sensor is based on determining that the respiratory pattern is currently indicating a maximum (126).

14. The XR apparatus of claim 12, wherein analyzing the respiratory pattern comprises determining minima of the respiratory pattern, and triggering the first image sensor is based on determining that the respiratory pattern is currently indicating a minimum.

15. The XR apparatus of any preceding claim, further comprising a display (128) configured to output an image captured by the first image sensor.
